# EUROPÄISCHE PATENTSCHRIFT

(11) **EP 3 724 115 B1**
(45) Veröffentlichungstag und Bekanntmachung des Hinweises auf die Patenterteilung: **04.01.2023**
(21) Anmeldenummer: 18816018.8
(22) Anmeldetag: 06.12.2018
(51) Int. Cl.: F03D 80/00, B66B 5/00, B66B 5/24, A61M 37/00, B66B 5/02

(54) **AUFSTIEGSHILFE FÜR EINE WINDENERGIEANLAGE**
LIFT SYSTEM FOR A WIND TURBINE
ASCENSEUR POUR UNE ÉOLIENNE

(30) Priorität: 11.12.2017 DE 102017129372
(43) Veröffentlichungstag der Anmeldung: 21.10.2020
(73) Patentinhaber: Wobben Properties GmbH, 26607 Aurich (DE)
(72) Erfinder: KLOTZ, Thomas, 39218 Schönebeck (DE)
(74) Vertreter: Eisenführ Speiser
(86) Internationale Anmeldenummer: PCT/EP2018/083859
(87) Internationale Veröffentlichungsnummer: WO 2019/115360

(56) Entgegenhaltungen:
- EP-A1- 3 181 503
- DE-A1-102016 109 859
- DE-B- 1 233 551
- JP-A- 2006 143 341
- Avanti: "Amendment to Avanti Service Lift, Model Shark - Chapter 5", , 22. Dezember 2016 (2016-12-22), XP055471589, Gefunden im Internet: URL:http://www.avanti-online.com/downloads /english [gefunden am 2018-04-30]

## Beschreibung

Die vorliegende Erfindung betrifft eine Aufstiegshilfe für eine Windenergieanlage, eine Windenergieanlage und ein Verfahren zum Testen einer Windenergieanlagen-Aufstiegshilfe.

Im Inneren eines Turms der Windenergieanlage wird typischerweise eine Aufstiegshilfe beispielsweise in Form eines Lifts vorgesehen, damit das Servicepersonal nach oben in die Gondel gelangen kann. Ferner können mittels der Aufstiegshilfe Material sowie Komponenten der Windenergieanlage nach oben transportiert werden.

Die Aufstiegshilfe wird typischerweise mittels eines Fahrseiles nach oben und unten bewegt. Als Sicherheitsmaßnahme ist typischerweise eine Fangvorrichtung vorgesehen, welche dann beispielsweise in ein redundantes Sicherheitsseil eingreift, wenn sich die Aufstiegshilfe unvorhergesehen nach unten bewegt.

Bevor eine Aufstiegshilfe durch das Servicepersonal verwendet wird, ist es aus sicherheitstechnischen Gründen vorgesehen, die Funktion der Fangvorrichtung zu prüfen. Dies wird typischerweise durch eine manuelle Betätigung des Sicherungsseiles durchgeführt. Da das Sicherungsseil inkl. der Fangvorrichtung jedoch auf dem Dach der Aufstiegshilfe vorgesehen ist, ist eine derartige manuelle Prüfung nur sehr schwierig durchzuführen.

In der prioritätsbegründenden deutschen Patentanmeldung hat das Deutsche Patent- und Markenamt die folgenden Dokumente recherchiert: DE 103 34 654 A1, DE 490 091 A, GB 2 263 681 A und EP 3 181 503 A1.

Es ist daher eine Aufgabe der vorliegenden Erfindung, eine Aufstiegshilfe für eine Windenergieanlage vorzusehen, welche eine verbesserte Überprüfung der Fangvorrichtung ermöglicht.

Diese Aufgabe wird durch eine Aufstiegshilfe für eine Windenergieanlage nach Anspruch 1 sowie durch eine Windenergieanlage nach Anspruch 10 gelöst.

Somit wird eine Aufstiegshilfe für eine Windenergieanlage vorgesehen, welche eine Kabine, eine Fangeinheit, die als Sicherungsmaßnahme mit einem Sicherungsseil zusammenwirkt und dazu ausgestaltet ist, zu verhindern, dass die Kabine sich unvorhergesehen nach unten bewegt, und eine Testeinheit zum Testen der Funktionsweise der Fangeinheit aufweist. Die Testeinheit weist eine Führungseinheit für das Sicherungsseil und eine Mitnahmeeinheit für das Sicherungsseil auf. Die Mitnahmeeinheit ist dazu geeignet das Sicherungsseil nach oben zu ziehen. Durch Betätigung der Mitnahmeeinheit wird das Sicherungsseil nach oben gezogen, was dazu führen kann, dass die Fangeinheit auslöst. Durch das Betätigen der Mitnahmeeinheit und das Hochziehen des Sicherungsseils kann damit ein Herunterfallen der Aufstiegshilfe simuliert werden, um die Funktionsweise der Fangeinheit testen zu können. Je schneller die Mitnahmeeinheit und damit das Sicherungsseil nach oben gezogen wird, desto schneller ist der simulierte Fall der Aufstiegshilfe.

Gemäß einem Aspekt der vorliegenden Erfindung weist die Führungseinheit eine Grundplatte auf. Die Mitnahmeeinheit weist einen Verschlussgegenhalter und einen Schließer auf. Die Testeinheit kann ferner ein Betätigungselement aufweisen, welches mit dem Schließer gekoppelt ist.

Gemäß einem weiteren Aspekt der vorliegenden Erfindung kann das Sicherungsseil durch Betätigung des Betätigungselementes mittels des Schließers und des Verschlussgegenhalters eingeklemmt und zusammen mit dem Schließer und dem Verschlussgegenhalter entlang der Grundplatte nach oben bewegt werden.

Gemäß einem weiteren Aspekt der vorliegenden Erfindung löst die Fangeinheit bei Betätigung des Betätigungselementes aus.

Gemäß einem weiteren Aspekt der vorliegenden Erfindung gibt der Schließer das Sicherungsseil frei, wenn das Betätigungselement losgelassen oder deaktiviert wird.

Somit wird eine Windenergieanlagen-Aufstiegshilfe mit einer Kabine, einer Fangeinheit, die als Sicherungsmaßnahme mit einem Sicherungsseil zusammenwirkt, und einer Testeinheit zum Testen der Funktionsweise der Fangeinheit vorgesehen. Die Testeinheit weist eine Grundplatte, einen Verschlussgegenhalter, einen Schließer und ein mit dem Schließer gekoppeltes Betätigungselement auf. Durch Betätigen des Betätigungselementes klemmen der Schließer und der Verschlussgegenhalter das Sicherungsseil ein und der Schließer und der Verschlussgegenhalter bewegen sich entlang der Grundplatte nach oben. Beim Loslassen oder Deaktivieren des Betätigungselementes gibt der Schließer das Sicherungsseil frei. Die Fangeinheit kann beim Betätigen des Betätigungselementes auf dem Weg P in der Testeinheit bereits auslösen.

Gemäß einem Aspekt der vorliegenden Erfindung ist das Betätigungselement als Seilzug ausgestaltet. Der Schließer ist mit einem Ende des Seilzuges gekoppelt und ein zweites Ende des Seilzugs reicht in die Kabine hinein. Durch Betätigung des Seilzuges klemmt der Schließer und der Verschlussgegenhalter das Sicherungsseil ein und der Schließer und der Verschlussgegenhalter werden entlang der Grundplatte nach oben bewegt. Die Fangeinheit kann beim Betätigen des Seilzugs auslösen. Beim Loslassen des Seilzugs gibt der Schließer das Sicherungsseil frei.

Gemäß einem Aspekt der vorliegenden Erfindung weist die Grundplatte mindestens ein Langloch auf. Der Schließer und der Verschlussgegenhalter sind auf einem Schlitten angeordnet, der bei Betätigung des Seilzugs entlang des mindestens einen Langlochs verfahrbar ist.

Gemäß einem weiteren Aspekt der vorliegenden Erfindung ist der Schließer über eine Torsionsfeder mit der Grundplatte gekoppelt und der Schließer wird in eine Ausgangsposition gedrückt.

Gemäß einem weiteren Aspekt der vorliegenden Erfindung kann das Betätigungselement als elektrisch aktivierbares Betätigungselement mit einem Aktuator ausgestaltet sein. Die Erfindung betrifft ebenfalls eine Windenergieanlage mit einer oben beschriebenen Aufstiegshilfe.

Die erfindungsgemäße Aufstiegshilfe weist typischerweise ein Dach auf, auf welchem eine Fangvorrichtung vorgesehen ist. Die Fangvorrichtung kann in ein redundantes Sicherheitsseil eingreifen. Eine Testeinheit zum Testen der Fangvorrichtung wird vorgesehen. Damit soll ein definiertes Testen der Fangvorrichtung ermöglicht werden.

Weitere Ausgestaltungen der Erfindung sind Gegenstand der Unteransprüche.

Vorteile und Ausführungsbeispiele der Erfindung werden nachstehend unter Bezugnahme auf die Zeichnung näher erläutert.
- Fig. 1: zeigt eine schematische Darstellung einer Windenergieanlage gemäß der Erfindung,
- Fig. 2: zeigt eine schematische Darstellung eines Abschnitts einer Aufstiegshilfe gemäß einem ersten Ausführungsbeispiel,
- Fig. 3: zeigt eine schematische Darstellung einer Fangvorrichtung für eine Aufstiegshilfe gemäß dem ersten Ausführungsbeispiel, und

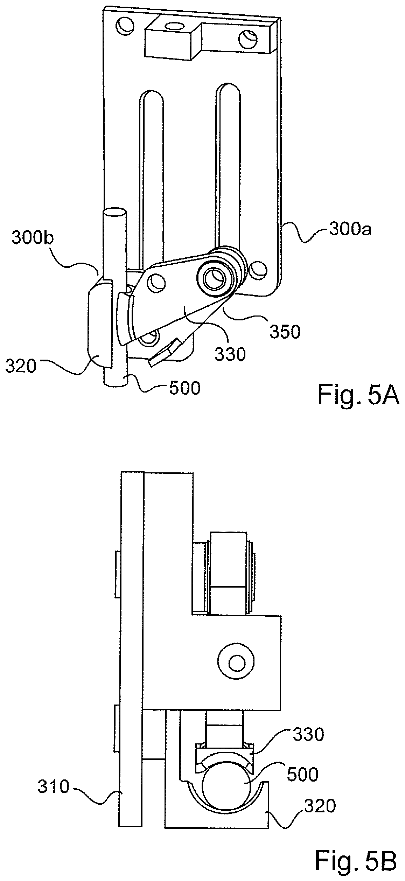

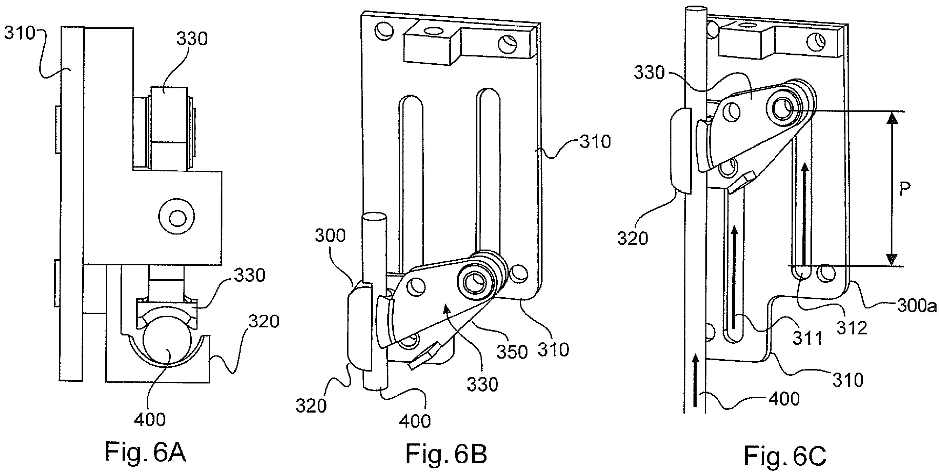

- Fig. 4A - 6C: zeigen jeweils eine schematische Darstellung einer Testeinheit für die Fangvorrichtung gemäß dem ersten Ausführungsbeispiel.

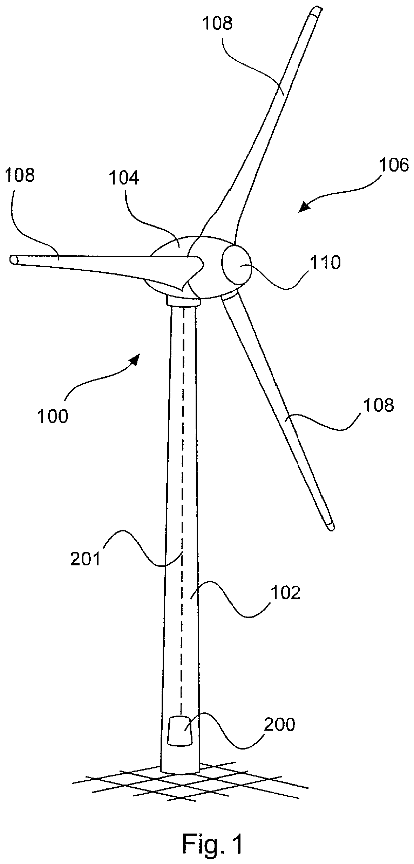

Fig. 1 zeigt eine schematische Darstellung einer Windenergieanlage gemäß der Erfindung. Die Windenergieanlage weist einen Turm 102 mit einer sich darauf befindlichen Gondel 104 auf. Die Gondel weist einen aerodynamischen Rotor 106 mit drei Rotorblättern 108 und einen Spinner 110 auf. Der aerodynamische Rotor 106 ist mit einem Rotor eines Generators (beispielsweise ein Synchrongenerator) gekoppelt, so dass eine Rotation des aerodynamischen Rotors 106 zu einer Rotation des Rotors des Generators führt, wodurch elektrische Energie erzeugt wird. Ein Pitchwinkel der Rotorblätter 108 ist mittels Pitchmotoren an den Rotorblattwurzeln einstellbar.

Im Inneren des Turms 102 ist eine Aufstiegshilfe 200 vorgesehen, damit Servicepersonal und Komponenten der Windenergieaniage vom Fuß des Turms 102 nach oben in die Gondel 104 transportiert werden können.

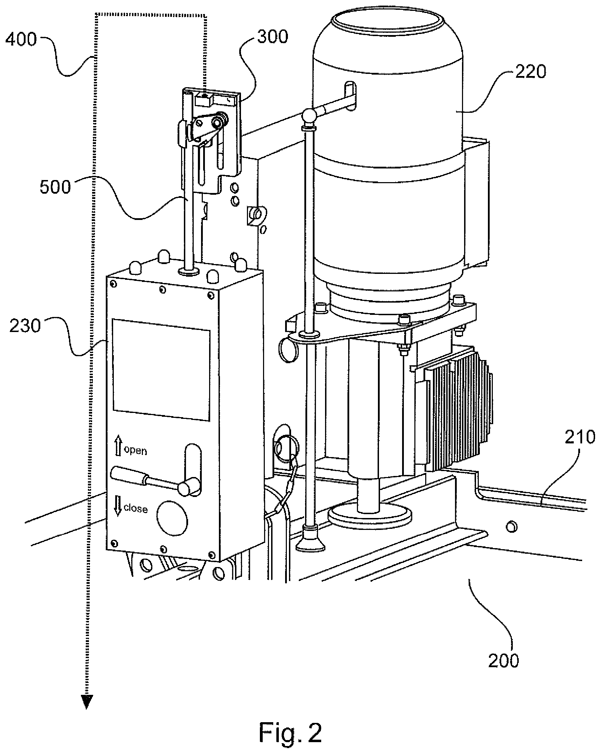

Fig. 2 zeigt eine schematische Darstellung eines Abschnitts einer Aufstiegshilfe gemäß einem ersten Ausführungsbeispiel. Die Aufstiegshilfe 200 weist einen Korb, Käfig, Rahmen oder eine Kabine 210 und einen Motor 220 auf, mittels welcher die Aufstiegshilfe über ein Fahrseil 201 nach oben oder unten befördert werden kann. Ferner weist die Aufstiegshilfe 200 eine Fangeinheit 230 auf, welche mit einem Sicherungsseil 500 zusammenwirkt und die Aufstiegshilfe 200 an dem Sicherungsseil 500 hält, wenn die Aufstiegshilfe 200 ungeplant nach unten fällt. Gemäß der Erfindung weist die Aufstiegshilfe 200 eine Testeinheit 300 auf, welche dazu dient, die Funktionsweise der Fangvorrichtung 230 zu testen.

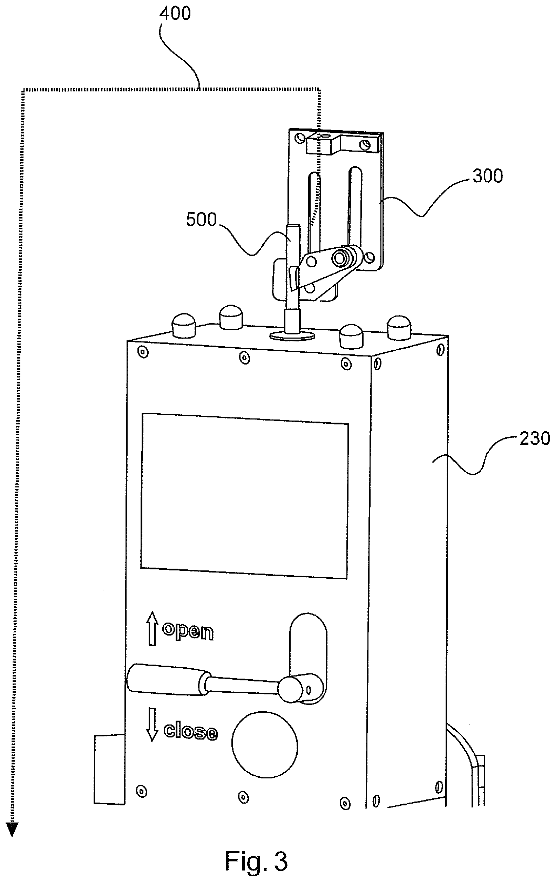

In Fig. 3 ist die Fangvorrichtung und die Testeinheit schematisch dargestellt. Das redundante Sicherheitsseil 500 ist mit der Fangvorrichtung 230 gekoppelt. Die Testeinheit 300 wird beispielsweise oberhalb der Fangvorrichtung 230 angeordnet und nimmt das Sicherheitsseil 500 auf.

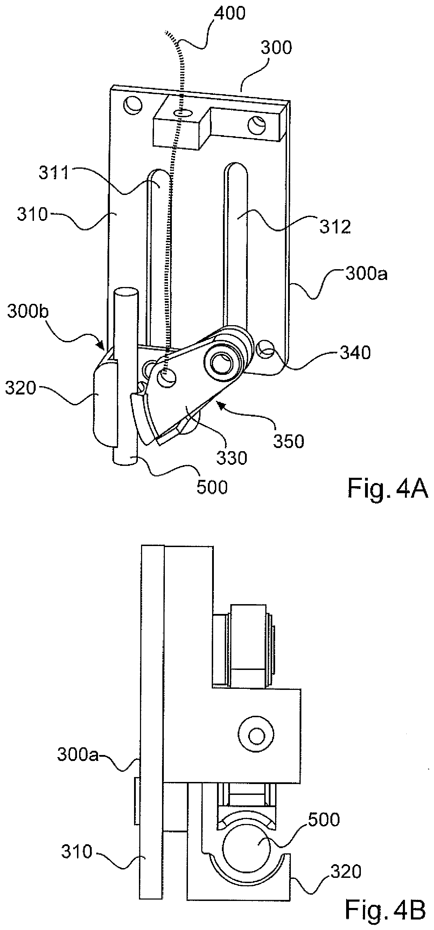

Fig. 4A bis 4C zeigen jeweils eine schematische Darstellung einer Testeinheit 300b für die Fangvorrichtung gemäß dem ersten Ausführungsbeispiel. Die Testeinheit 300 weist eine Führungseinheit 300a mit einer Grundplatte 310 mit zwei Ausnehmungen oder Langlöchern 311, 312, und eine Mitnahmeeinheit 300b mit einem Verschlussgegenhalter 320, einem Schließer 330 und optional einer Torsionsfeder 340 auf. Ein Betätigungselement (z.B. ein Seilzug 400) ist mit der Mitnahmeeinheit und z. B. dem Schließer 330 gekoppelt.

Die Mitnahmeeinheit 300b (der Verschlussgegenhalter 320 und der Schließer 330) kann auf einem Schlitten 350 vorgesehen sein, der entlang der Langlöcher 311, 312 verfahrbar ist.

Im normalen Fahrbetrieb 330 ist die Mitnahmeeinheit 300, z. B. der Schließer 330, nicht in Kontakt mit dem Sicherungsseil 500 und die Aufstiegshilfe 200 kann ohne Behinderung nach oben oder unten innerhalb des Turms 102 verfahren werden.

Gemäß der Erfindung wird die Grundplatte 310 der Testeinheit 300 fest in oder an der Aufstiegshilfe 200 verschraubt. Durch Betätigung des Seilzugs 400 schließt der Schließer 330 entgegen der Torsionskraft der Feder 340 und das Sicherungsseil 500 wird in der Mitnahmeeinheit, d.h. zwischen Verschlussgegenhalter 320 und Schließer 330 eingeklemmt. Beim weiteren Ziehen kann das Sicherungsseil 500 durch die Mitnahmeeinheit 300b mitgenommen werden und die Mitnahmeeinheit der Verschlussgegenhalter 320 wird zusammen mit dem Schließer entlang der Grundplatte 310 nach oben bewegt. Die Länge der Ausnehmung bzw. der Langlöcher 311, 312 bestimmt den maximalen Prüfweg P. Hierbei sollte die Fangvorrichtung 230 auslösen. Anschließend kann der Schließer 330 wieder geöffnet werden und der Verschlussgegenhalter 320 kann wieder in seine Ausgangsposition nach unten verfahren. Dies geschieht, indem der Schließer 330 indirekt über den Seilzug 400 entlastet wird, und die Torsionsfeder 340 öffnet den Schließer 330 dann. Der Seilzug 400 kann in das Innere der Aufstiegshilfe hineinragen, so dass ein Servicemitarbeiter den Seilzug betätigen kann, um die Fangvorrichtung mittels der Testeinheit 300 zu kontrollieren.

Durch Betätigung des Seilzuges 400 wird der Schließer 330 betätigt, so dass der Schließer 330 an dem Sicherheitsseil 500 anliegt. Das Sicherheitsseil 500 wird hierbei zwischen dem Verschlussgegenhalter 320 und dem Schließer 330 fixiert. Wenn der Seilzug 400 weiter betätigt wird, dann wird das Sicherheitsseil 500 (welches zwischen dem Verschlussgegenhalter und dem Schließer fixiert ist) mitgenommen und die Einheit aus Verschlussgegenhalter und Schließer (nämlich der Schlitten) wird entlang der Langlöcher 311, 312 nach oben bewegt. Wenn dies ausreichend schnell erfolgt, dann wird die Fangvorrichtung 230 ausgelöst, bevor der Schlitten den maximalen Prüfweg P zurückgelegt hat. Anschließend kann der Seilzug über die Feder 340 in seine Ausgangslage gebracht werden und der Schlitten 350 bewegt sich nach unten und der Schließer 330 öffnet sich, so dass das Sicherheitsseil 500 wieder frei ist und nicht mehr fixiert ist. Somit kann mittels der Testeinheit 300 die Funktion der Fangvorrichtung überprüft werden. Nach Entriegelung der Fangvorrichtung 230 kann der Fahrbetrieb der Aufstiegshilfe 200 aufgenommen werden.

Gemäß einem Aspekt der vorliegenden Erfindung kann die Sicherheitsseilmitnahme über einen Schließer beispielsweise in Form eines konischen Mitnehmers erfolgen. Alternativ dazu kann eine elektrische Auslösung vorgesehen werden.

Gemäß einem Aspekt der vorliegenden Erfindung könnte im Falle einer elektrischen Auslösung ein Beschleunigungswert eingestellt werden, so dass die Fangvorrichtung für alle Auslösevariablen geprüft werden kann.

## Patentansprüche

1. Aufstiegshilfe für eine Windenergieanlage, mit
einer Kabine (210),
einer Fangeinheit (230), die als Sicherungsmaßnahme mit einem Sicherungsseil (500) zusammenwirkt und dazu ausgestaltet ist, zu verhindern, dass die Kabine (210) sich unvorhergesehen nach unten bewegt, und
einer Testeinheit (300) zum Testen der Funktionsweise der Fangeinheit (230),
wobei die Testeinheit (300) eine Führungseinheit (300a) für das Sicherungsseil (500) aufweist,
**dadurch gekennzeichnet, dass** die Testeinheit (300) eine Mitnahmeeinheit (300b) für das Sicherungsseil (500) aufweist, welches dazu geeignet ist, das Sicherungsseil (500) nach oben zu ziehen.

2. Aufstiegshilfe nach Anspruch 1, wobei
die Führungseinheit (300a) eine Grundplatte (310) aufweist,
wobei die Mitnahmeeinheit (300b) einen Verschlussgegenhalter (320) und einen Schließer (330) aufweist und ein mit dem Schließer (330) gekoppeltes Betätigungselement (400) aufweist.

3. Aufstiegshilfe nach Anspruch 2, wobei
durch Betätigung des Betätigungselementes (400) der Schließer (330) und der Verschlussgegenhalter (320) das Sicherungsseil (500) einklemmt und der Schließer (330) und der Verschlussgegenhalter (320) entlang der Grundplatte (310) nach oben bewegt wird.

4. Aufstiegshilfe nach Anspruch 2 oder 3, wobei
die Fangeinheit (230) bei der Betätigung des Betätigungselementes (400) auslöst.

5. Aufstiegshilfe nach Anspruch 4, wobei
beim Loslassen oder Deaktivieren des Betätigungselementes (400) der Schließer (310) das Sicherungsseil (500) freigibt.

6. Aufstiegshilfe (200) nach einem der Ansprüche 2 bis 4, wobei
das Betätigungselement (400) als Seilzug ausgestaltet ist,
wobei der Schließer (330) mit einem Ende eines Seilzugs (400) gekoppelt ist, wobei ein zweites Ende des Seilzugs (400) in die Kabine (210) hineinreicht, wobei durch Betätigung des Seilzugs (400) der Schließer (330) und der Verschlussgegenhalter (320) das Sicherungsseil (500) einklemmt und der Schließer (330) und der Verschlussgegenhalter (320) entlang der Grundplatte (310) nach oben bewegt wird,
wobei die Fangeinheit (230) bei der Betätigung des Betätigungselementes (400) auslöst,
wobei beim Loslassen des Seilzugs (400) der Schließer (310) das Sicherungsseil (500) freigibt.

7. Aufstiegshilfe nach Anspruch 6, wobei
die Grundplatte (310) mindestens ein Langloch (311, 312) aufweist,
wobei der Schließer (330) und der Verschlussgegenhalter (320) auf einem Schlitten (350) angeordnet sind, der bei Betätigung des Seilzugs (400) entlang des mindestens einen Langlochs (311, 312) verfahrbar ist.

8. Aufstiegshilfe nach einem der Ansprüche 2 bis 6, wobei
der Schließer (330) über eine Torsionsfeder (340) mit der Grundplatte gekoppelt ist und den Schließer (330) in eine Ausgangsposition drückt.

9. Aufstiegshilfe nach einem der Ansprüche 2 bis 6, wobei
das Betätigungselement (400) als ein elektrisch aktivierbares Betätigungselement mit einem Aktuator ausgestaltet ist.

10. Windenergieanlage, mit
einer Aufstiegshilfe nach einem der Ansprüche 1 bis 9.

11. Verfahren zum Testen einer Fangeinheit (230) einer Windenergieanlagen-Aufstiegshilfe, wobei die Aufstiegshilfe eine Kabine (210), eine Fangeinheit (230) und eine Testeinheit (300) aufweist,
wobei die Fangeinheit (230) mit einem Sicherungsseil (500) zusammenwirkt, um zu verhindern, dass die Kabine (210) sich unvorhergesehen nach unten bewegt, wobei die Testeinheit (300) an der Kabine (210) befestigt ist, **gekennzeichnet durch** den Schritt:
Ziehen des Sicherungsseils nach oben mittels einer Mitnahmeeinheit (300b), so dass die Fangeinheit (230) ausgelöst wird.

## Claims

1. An ascension aid for a wind turbine comprising
a cabin (210),
a catch unit (230) which co-operates with a securing cable (500) as a securing means and is adapted to prevent the cabin (210) from moving unexpectedly downwardly, and
a testing unit (300) for testing the functionality of the catch unit (230),
wherein the testing unit (300) has a guide unit (300a) for the securing cable (500) **characterized in that** the testing unit (300) comprises an entrainment unit (300b) for the securing cable (500), which is suitable for pulling the securing cable (500) upwardly.

2. An ascension aid according to claim 1 wherein
the guide unit (300a) has a base plate (310),
wherein the entrainment unit (300b) has a closure counterpart holder (320) and a closing member (330) and has an actuating element (400) coupled to the closing member (330).

3. An ascension aid according to claim 2 wherein
by actuation of the actuating element (400) the closing member (330) and the closure counterpart holder (320) clamp the securing cable (500) and the closing member (330) and the closure counterpart holder (320) are moved upwardly along the base plate (310).

4. An ascension aid according to claim 2 or claim 3 wherein
the catch unit (230) trips upon actuation of the actuating element (400).

5. An ascension aid according to claim 4 wherein
upon release or de-activation of the actuating element (400) the closing member (310) releases the securing cable (500).

6. An ascension aid (200) according to one of claims 2 to 4 wherein
the actuating element (400) is in the form of a cable pull,
wherein the closing member (330) is coupled to one end of the cable pull (400), wherein a second end of the cable pull (400) extends into the cabin (210), wherein by actuation of the cable pull (400) the closing member (330) and the closure counterpart holder (320) clamp the securing cable (500) and the closing member (330) and the closure counterpart holder (320) are moved upwardly along the base plate (310),
wherein the catch unit (230) trips upon actuation of the actuating element (400),
wherein upon release the cable pull (400) the closing member (310) releases the securing cable (500).

7. An ascension aid according to claim 6 wherein
the base plate (310) has at least one slot (311, 312),
wherein the closing member (330) and the closure counterpart holder (320) are arranged on a slider (350) which upon actuation of the cable pull (400) is displaceable along the at least one slot (311, 312).

8. An ascension aid according to one of claims 2 to 6 wherein
the closing member (330) is coupled to the base plate by way of a torsion spring (340) and urges the closing member (330) into a starting position.

9. An ascension aid according to one of claims 2 to 6 wherein
the actuating element (400) is in the form of an electrically activatable actuating element having an actuator.

10. A wind turbine comprising
an ascension aid according to one of claims 1 to 9.

11. A method of testing a catch unit (230) of a wind turbine ascension aid, wherein the ascension aid has a cabin (210), a catch unit (230) and a testing unit (300),
wherein the catch unit (230) co-operates with a securing cable (500) to prevent the cabin (210) from moving unexpectedly downwardly, wherein the testing unit (300) is fixed to the cabin (210), **characterized by** the step:
pulling the securing cable upwardly by means of the entrainment unit (300b) so that the catch unit (230) is tripped.

## Revendications

1. Aide à la montée pour une éolienne, avec
une cabine (210),
une unité de retenue (230), qui coopère en tant que mesure de sécurité avec un câble de sécurité (500) et est conçue pour empêcher que la cabine (210) ne se déplace vers le bas de manière imprévue, et
une unité de test (300) pour tester le fonctionnement de l'unité de retenue (230),
dans laquelle l'unité de test (300) présente une unité de guidage (300a) pour le câble de sécurité (500),
**caractérisée en ce que** l'unité de test (300) présente une unité d'entraînement (300b) pour le câble de sécurité (500), laquelle est adaptée pour tirer le câble de sécurité (500) vers le haut.

2. Aide à la montée selon la revendication 1, dans laquelle
l'unité de guidage (300a) présente une plaque de base (310),
dans laquelle l'unité d'entraînement (300b) présente un contre-support de fermeture (320) et un contact à fermeture (330) et présente un élément d'actionnement (400) accouplé au contact à fermeture (330) .

3. Aide à la montée selon la revendication 2, dans laquelle
par actionnement de l'élément d'actionnement (400) le contact à fermeture (330) et le contre-support de fermeture (320) coincent le câble de sécurité (500) et le contact à fermeture (330) et le contre-support de fermeture (320) sont déplacés vers le haut le long de la plaque de base (310).

4. Aide à la montée selon la revendication 2 ou 3, dans laquelle
l'unité de retenue (230) se déclenche lors de l'actionnement de l'élément d'actionnement (400).

5. Aide à la montée selon la revendication 4, dans laquelle
lors du relâchement ou de la désactivation de l'élément d'actionnement (400) le contact à fermeture (310) libère le câble de sécurité (500).

6. Aide à la montée (200) selon l'une quelconque des revendications 2 à 4, dans laquelle
l'élément d'actionnement (400) est conçu en tant que câble de traction,
dans laquelle le contact à fermeture (330) est accouplé à une extrémité d'un câble de traction (400), dans laquelle
une deuxième extrémité du câble de traction (400) entre dans la cabine (210), dans laquelle par actionnement du câble de traction (400) le contact à fermeture (330) et le contre-support de fermeture (320) coincent le câble de sécurité (500) et le contact à fermeture (330) et le contre-support de fermeture (320) sont déplacés vers le haut le long de la plaque de base (310),
dans laquelle l'unité de retenue (230) se déclenche lors de l'actionnement de l'élément d'actionnement (400),
dans laquelle lors du relâchement du câble de traction (400) le contact à fermeture (310) libère le câble de sécurité (500).

7. Aide à la montée selon la revendication 6, dans laquelle
la plaque de base (310) présente au moins un trou oblong (311, 312),
dans laquelle le contact à fermeture (330) et le contre-support de fermeture (320) sont disposés sur un coulisseau (350), qui est déplaçable le long du au moins un trou oblong (311, 312) lors de l'actionnement du câble de traction (400).

8. Aide à la montée selon l'une quelconque des revendications 2 à 6, dans laquelle
le contact à fermeture (330) est accouplé à la plaque de base par l'intermédiaire d'un ressort de torsion (340) et presse le contact à fermeture (330) dans une position de départ.

9. Aide à la montée selon l'une quelconque des revendications 2 à 6, dans laquelle
l'élément d'actionnement (400) est conçu en tant qu'élément d'actionnement pouvant être activé électriquement avec un actionneur.

10. Eolienne, avec
une aide à la montée selon l'une quelconque des revendications 1 à 9.

11. Procédé pour tester une unité de retenue (230) d'une aide à la montée d'éolienne, dans lequel l'aide à la montée présente une cabine (210), une unité de retenue (230) et une unité de test (300),
dans lequel l'unité de retenue (230) coopère avec un câble de sécurité (500), afin d'empêcher que la cabine (210) ne se déplace de manière imprévue vers le bas, dans lequel l'unité de test (300) est fixée sur la cabine (210), **caractérisé par** l'étape :
de traction du câble de sécurité vers le haut au moyen d'une unité d'entraînement (300b), de sorte que l'unité de retenue (230) est déclenchée.
